# FASCICULE DE BREVET EUROPEEN

(11) **EP 0 512 883 B1**
(45) Date de publication et mention de la délivrance du brevet: **06.08.1997**
(21) Numéro de dépôt: 92401157.0
(22) Date de dépôt: 23.04.1992
(51) Int. Cl.: A61K 35/16, A61K 38/36

(54) **Procédé de préparation d'un concentré de facteur XI de la coagulation sanguine à haute activité spécifique, approprié à un usage thérapeutique**
Verfahren zur Herstellung eines Konzentrates von Blutgerinnungsfaktor XI mit hoher spezifischer Aktivität zur therapeutischen Verwendung
Process for preparing a concentrate of blood coagulation factor XI with high specific activity, appropriate for therapeutic use

(30) Priorité: 07.05.1991 FR 9105572
(43) Date de publication de la demande: 11.11.1992
(73) Titulaire: ASSOCIATION POUR L'ESSOR DE LA TRANSFUSION SANGUINE DANS LA REGION DU NORD, F-59012 Lille (FR)
(72) Inventeur: Burnouf, née Radosevich, Miryana, F-59136 Wavrin (FR); Dernis, Dominique, F-59520 Marquette-Lez-Lille (FR)
(74) Mandataire: Lepeudry-Gautherat, Thérèse

(56) Documents cités:
- BIOLOGICAL ABSTRACTS, Vol. 65, Réf.12893 (1978); BOUMA et al.: 'Human blood coagulation factor XI: Purification, properties and mechanism of activa tion by activated factor XII.'; J. Biol. Chem. 252(18), 6432-6437 (1977)
- J. Biol. Chem., vol. 252, no. 18, pp. 6432-6437 (1977)

## Description

L'invention concerne un procédé de préparation d'un concentré de Facteur XI plasmatique humain à très haute activité spécifique destiné à un usage thérapeutique.

Le Facteur XI ou précurseur de la thromboplastine plasmatique est une glycoprotéine qui fait partie de la phase de contact, dans le mécanisme de l'hémostase, par son action activatrice du Facteur IX et, d'autre part, du système fibrinolytique par son action activatrice du plasminogène.

La déficience en Facteur XI est héréditaire et se transmet comme un caractère autosome récessif. C'est une déficience rare mais répandue chez certaines populations d'origine moyen-orientale.

Comme pour d'autres facteurs dont la déficience est rare (Facteur V, XIII, X) des produits thérapeutiques purifiés à partir de plasma humain sont encore rares ou inexistants et la seule thérapie de remplacement est effectuée avec du plasma total ou la fraction surnageante du plasma cryoprécipité mais ceci entraîne l'injection simultanée de quantités inutiles des autres protéines du plasma et donc un risque de réactions secondaires diverses importantes après des injections multiples.

La purification du Facteur XI, à l'échelle expérimentale, n'a pu être réalisée que difficilement et à condition d'utiliser de puissants inhibiteurs, ce qui suggère que cette molécule est très labile. La purification a été réalisée par une succession de 4 ou 5 étapes de chromatographie d'échange d'ions et d'affinité, soit à partir de plasma (Bouma et Griffin, 1977, J. Biol. Chem. 252, 6432-6437) soit à partir des plaquettes (Schiffman et Yeh, 1990, Thromb. Res. 60, 87-97). Une préparation de Facteur XI bovin de haute pureté a aussi été purifiée à partir de 20 litres de plasma, par précipitation et chromatographies, l'ensemble des étapes étant réalisé en 9 jours environ (Koide et al., 1975, dans : Methods in Enzymology - Blood clotting enzymes - pp 65-73). Une seule préparation de qualité compatible avec une utilisation thérapeutique a été décrite (Winkelman et al. 1988, Internat. Congress ISBT-BBTS London) par adsorption sur héparine-sépharose après récupération des Facteurs VIII et IX, mais son activité spécifique ne dépasse pas 5 U/mg de protéine et elle contient une grande quantité d'AT III résiduelle.

La Demanderesse a donc cherché à mettre au point un nouveau procédé de purification adapté à de très grands volumes de plasma et qui permet d'obtenir en un petit nombre d'étapes faciles à réaliser industriellement, un concentré de Facteur XI de qualité appropriée à un usage thérapeutique.

Ainsi la présente invention concerne un concentré de Facteur XI humain dont la préparation ne comprend que deux étapes : la première est une filtration-adsorption qui retient assez sélectivement le Facteur XI ; après désorption de celui-ci, il est soumis à la seconde étape qui est une chromatographie sur résine échangeuse de cations.

La préparation de Facteur XI est soumise à un traitement classique d'inactivation virale par solvant-détergent avant l'étape de chromatographie, cette dernière permettant d'éliminer totalement les produits résiduels de cette étape de décontamination.

Le procédé selon la présente invention permet la production de Facteur XI à usage thérapeutique à partir de 1.100 litres de plasma environ, dans un temps assez court (environ 28 heures) qui comprend les 8 heures de traitement d'inactivation virale.

Le procédé de purification selon la présente invention s'applique à du surnageant de plasma cryoprécipité et peut être adapté à des volumes de 1.000 litres à 1.200 litres.

La première étape de purification est une filtration sur une batterie de 3 cartouches de filtres de porosité comprise entre 0,5 et 2 µ et essentiellement chargés négativement. (filtres Zeta plus Ⓜ, fournis par Cuno, Process Filtration Products, filiale de Commercial Intertech Corp. USA, et décrits dans les brevets US 4,783,262 et 4,859,340, ci-après dénommés "filtres Cuno". Ces filtres sont constitués de cellulose purifiée, de perlites et d'une faible quantité de résine chargée positivement. D'autres systèmes de filtres disponibles dans le commerce peuvent également être utilisés.

Les filtres sont rincés en tampon citrate/phosphate comprenant du citrate de sodium, du phosphate disodique, du phosphate de potassium, du chlorure de sodium et de l'EDTA-disodique et ajusté à un pH compris entre 5,5 et 6,5 et, préférentiellement, à pH 6. Cette étape de filtration élimine une grande partie des protéines plasmatiques.

Le Facteur XI qui est resté adsorbé sur les couches de filtres, en raison de leur charge négative, en est désorbé par une augmentation de la force ionique du tampon par un ajustement de la concentration finale en chlorure de sodium de 0,5 à 2 M. Ce dernier tampon est également additionné d'une faible quantité d'antithrombine III (AT III), de 0,1 à 0,2 U/ml, pour protéger le Facteur XI contre l'action des protéases.

La fraction désorbée, dialysée et concentrée est ensuite injectée sur une colonne de chromatographie en résine échangeuse de cations, plus particulièrement en gel de sulfate de sépharose, équilibrée en tampon composé de citrate de sodium, de chlorure de sodium, de lysine et d'arginine, à un pH de 5,5 à 6,5 et de préférence à pH 6.

Cette colonne laisse passer les protéines contaminantes subsistantes et les agents d'inactivation virale.

Le gel de sulfate de sépharose présente, de manière inattendue, une très haute capacité de rétention du Facteur XI (de 300 à 450 U/ml de gel) ce qui permet d'éviter une étape ultérieure d'ultrafiltration qui entraînerait une perte de rendement. De plus la fixation au sulfate de sépharose permet une élution par du tampon pratiquement physiologique alors que les autres résines (décrites plus haut) qui sont à base de groupements sulfopropyle nécessitent des conditions d'élution plus drastiques.

Après rinçage de la colonne par du tampon citrate/phosphate comprenant du citrate de sodium, du phosphate disodique, du phosphate de potassium, du chlorure de sodium, de la lysine et de l'arginine, ajustée à un pH compris entre 6,1 et 6,9 et préférentiellement à pH 6,5, le Facteur XI est désorbé par une augmentation du pH du tampon entre 7 et 8 et, de préférence à 7,5 et de la concentration de chlorure de sodium à 0,15 à 0,20 M et de préférence à 0,17 M

Dès son élution, le Facteur XI est stabilisé par l'addition de 0,5 à 3 U/ml d'AT III de haute pureté et de 0,5 à 4 U/ml d'héparine. Puis la solution est stérilisée par filtration, conditionnée et lyophilisée.

Le facteur de purification du présent procédé est supérieur à 10000 par rapport au plasma de départ.

Le Facteur XI obtenu par le procédé selon la présente invention présente une activité spécifique au moins égale à 100 U/mg de protéines.

La haute pureté du Facteur XI obtenu est démontrée par électrophorèse sur gel de polyacrylamide-SDS, par des analyses biochimiques et son innocuité par des tests biologiques sur animaux.

Le concentré de Facteur XI obtenu par le procédé selon la présente invention est donc particulièrement bien adapté à un usage thérapeutique, en particulier comme thérapie de remplacement dans les cas de déficience congénitale ou acquise en Facteur XI.

L'exemple suivant illustre un mode de réalisation de la présente invention sans toutefois en limiter la portée.

### Exemple

### - Matériau de départ

Chaque lot de Facteur XI est préparé à partir d'un volume d'environ 1000 litres de surnageant de cryoprécipité de plasma humain.

### - Première étape de purification

Le surnageant du cryoprécipité est passé sur des cartouches de "filtres Cuno" disposées en batteries de trois. On utilise soit des filtres de 0,5-1 µ (de type 50 S) soit des filtres de 1-2 µ (de type 30 S).

Après élimination du filtrat qui contient une majorité des protéines du surnageant du cryoprécipité, les cartouches sont lavées avec du tampon citrate/phosphate comprenant du citrate de sodium 5 mM, du phosphate disodique 5 mM, du phosphate de potassium 5 mM, du chlorure de sodium 0,065 M et de l'EDTA-disodique 0,5 mM, et ajusté à un pH de 6 avec de l'acide citrique.

On observe une adsorption relativement sélective du Facteur XI sur les couches de filtres.

Le Facteur XI est désorbé des filtres par une augmentation de la force ionique du tampon de lavage par un ajustement de la concentration en NaCl à 1 M. On y ajoute également 0,2 U/ml d'AT III pour protéger le Facteur XI de l'action des protéases plasmatiques résiduelles ; l'EDTA du tampon participe également à cette action protectrice.

La solution de Facteur XI ainsi récupérée est concentrée 30 fois et dialysée pour éliminer l'EDTA, à l'aide d'un système à ultrafiltration Millipore Ⓜ constitué de 10 cassettes à membranes 10 K.

Le tampon de dialyse est composé de citrate de sodium 5 mM, de chlorure de sodium 0,14 M, de L-lysine 5,5 mM et de L-arginine 20 mM, et ajusté à pH 6.

La solution dialysée est passée sur un filtre 0,45 µ DSLK2NLP (PALL Ⓜ) pour clarifier la solution et éventuelement éliminer des contaminants bactériens.

### - Traitement d'inactivation virale

La solution contenant le Facteur XI est soumise à un traitement par solvant-détergent connu pour son efficacité contre les virus à enveloppe lipidique (Horowitz et al. 1985, Transfusion 25, 516-522) et qui comprend une incubation de 8 heures à 25°C en présence de 0,3 % de tri-n-butyl-phosphate (TnBP) et de 1 % de Tween 80.

### - Deuxième étape de purification

On utilise une colonne de chromatographie en résine échangeuse de cations, plus particulièrement le gel de "sulfate-sepharose-fast flow" Ⓜ (fourni par Pharmacia, Uppsala-Suède).

La colonne est équilibrée avec le tampon de dialyse décrit plus haut.

Après avoir chargé la solution protéique sur la colonne, celle-ci est rincée avec 10 à 15 volumes de tampon de rinçage pour éliminer les protéines faiblement adsorbées et les agents d'inactivation virale. Ce tampon de rinçage comprend du citrate de sodium 10 mM, du phosphate disodique 5 mM, du phosphate de potassium 5 mM, du chlorure de sodium 0,12 M, de la lysine 27 mM et de l'arginine 11,5 mM, à pH 6,5

La vitesse de flux linéaire des tampons d'équilibrage, de lavage et d'élution est de 50 cm/h.

Le Facteur XI est élué de la colonne par une augmentation du pH du tampon à 7,5 et une augmentation de la concentration en NaCl à 0,17 M.

Dès son élution le Facteur XI est additionné de 2 U/ml d'AT III (c'est-à-dire environ 1,5 à 2,5 % du taux de FXI) et de 5 U/ml d'héparine (c'est-à-dire 1,5 à 2,5 % du taux de FXI) pour le stabiliser. (Les deux produits sont de haute pureté et de qualité injectable à l'homme).

La solution de Facteur XI ainsi préparée est stérilisée par filtration sur filtre 0,22 µ DSLK1NFZP (PALL Ⓜ), distribuée (10 ml par flacon) et lyophilisée.

### - Analyses biochimiques et biologiques du concentré de Facteur XI

Six lots successifs ont été analysés.

L'activité spécifique du Facteur XI est de 130 à 150 U/mg de protéines (c'est-à-dire FXI + AT III). Avant l'addition d'AT III, l'activité spécifique est de 210 U/mg.

Le Facteur XI ajusté à environ 100-120 U/ml avant lyophilisation présente une activité coagulante de 90 à 110 U/ml.

Les tableaux suivants montrent les caractéristiques du produit purifié.

**TABLEAU I**

| Récupération du Facteur XI et pureté du produit (moyenne de 6 lots) | | | |
|---|---|---|---|
| Fractions | % de récupération | activité spécifique en U/mg | facteur de purification |
| éluat des filtres Cuno | 37 | 12 | ≃ 800 |
| après traitement solvant-détergent | 98 | 12 | |
| éluat de la chromatographie sur S-sepharose | 62 | 210 | ≃ 14.000 |

**TABLEAU II**

| Caractéristiques du Facteur XI lyophilisé (moyenne de 6 lots) | |
|---|---|
| Contenu en protéines (g/l) | 0,7-1,1 |
| Facteur XI (U/ml) | 90-110 |
| Activité spécifique (U/mg) (après addition d'AT III) | 130-150 |
| Inhibiteur Cl* | 0,34 |
| Fibronectine* | 0,46 |
| alpha 2-macroglobuline* | 0,13 |
| IgG* | 0,18 |
| Albumine | < 0,0006 |
| Fibrinogène | < 0,002 |
| NAPPT (dilution 1/10) | > 600'' |
| NAPPT (dilution 1/100) | 221'' |
| NAPPT (1/10)** | 166'' |
| NAPPT (1/100)** | 216'' |
| FCT (20° C) | > 24 h |
| FCT (37° C) | > 6 h |

| | |
|---|---|
| * Exprimé en mg/1000 U F XI | |
| ** Dilutions testées après neutralisation de l'héparine | |

La faible quantité de contaminants protéiques (à l'exception de l'AT III ajoutée volontairement) est confirmée par immunonéphélométrie.

L'électrophorèse sur gel de polyacrylamide-SDS montre une seule bande majeure à 160 KDa et une petite bande à 62 KDa qui correspond à l'AT III. Après réduction des protéines par le β-mercapto-éthanol, aucune bande n'est détectable dans la région 50-30 KDa ce qui démontre que les molécules de Facteur XI n'ont pas été activées au cours du procédé de purification (Bouma et Griffin, 1986, Blood coagulation Ed. Hemker - p. 103-128).

L'absence de contamination résiduelle par des facteurs de la coagulation et des constituants du système kinine est soigneusement contrôlée selon les méthodes classiques.

Après reconstitution du produit lyophilisé des tests classiques de tolérance sur animaux sont exécutés :
- test de thrombogénicité sur lapins
- test d'hypotension sur rats
- test de toxicité sur souris.

Les tests sur lapins démontrent que le produit n'est pas thrombogène puisque la DE 50 (dose efficace 50) est supérieure à 1000 U FXI/kg alors que cette même valeur est de 40 à 60 U/kg pour un concentré de PPSB qui est donc beaucoup plus thrombogène, et peur entraîner effectivement des phénomènes de thromboses et de coagulation intravasculaire disséminée à forte dose chez l'homme.

Le concentré n'induit pas de phénomènes d'hypotension quand il est injecté par voie intraveineuse chez le rat à la dose de 50 U FXI/kg. Ce modèle animal est très sensible à la présence de composants plasmatiques à propriétés vasoactives et démontre l'absence de ces composants dans le concentré de facteur XI obtenu par le procédé décrit.

Injecté par voie intraveineuse chez la souris à une forte dose (2500 U/kg) il n'induit aucune létalité ni aucune perturbation du comportement sur une période de 7 jours.

## Revendications

1. Procédé de préparation d'un concentré de Facteur XI humain de qualité appropriée à un usage thérapeutique, caractérisé en ce qu'il comprend une étape de filtration-adsorption et une étape unique de chromatographie sur résine échangeuse de cations.

2. Procédé selon la revendication 1, caractérisé en ce que l'étape de filtration-adsorption est réalisée sur une série de cartouches de filtres de porosité de 0,5 à 2µ, composés de cellulose et de perlites et portant des charges négatives ainsi que d'une faible quantité de résine chargée positivement.

3. Procédé selon la revendication 1 ou 2, caractérisé en ce que le filtre est rincé en tampon comprenant du citrate de sodium, du phosphate disodique, du phosphate de potassium, du chlorure de sodium et de l'EDTA-disodique, ajusté à un pH de 5,5 à 6,5.

4. Procédé selon l'une quelconque des revendications 1 à 3, caractérisé en ce que le Facteur XI est désorbé des filtres par une augmentation de la force ionique du tampon.

5. Procédé selon la revendication 4, caractérisé en ce qu'on augmente la force ionique du tampon en portant la concentration en chlorure de sodium à 0,5 à 2 M.

6. Procédé selon la revendication 5, caractérisé en ce que le tampon d'élution est additionné de 0,1 à 0,2 U/ml d'antithrombine III.

7. Procédé selon l'une quelconque des revendications 1 à 6, caractérisé en ce que la solution de Facteur XI désorbée est dialysée, concentrée et soumise à un traitement d'inactivation virale.

8. Procédé selon l'une quelconque des revendications 1 à 7, caractérisé en ce que la solution de Facteur XI est soumise à une chromatographie sur résine échangeuse de cations.

9. Procédé selon la revendication 8, caractérisé en ce que la résine est du sulfate de sépharose.

10. Procédé selon l'une quelconque des revendications 1 à 9, caractérisé en ce que la colonne de chromatographie est équilibrée en tampon comprenant du citrate de sodium, du chlorure de sodium, de la lysine et de l'arginine et ajusté à un pH de 5,5 à 6,5.

11. Procédé selon l'une quelconque des revendications 1 à 10, caractérisé en ce qu'après la charge de la colonne par la solution de Facteur XI, celle-ci est rincée par du tampon comprenant du citrate de sodium, du phosphate disodique, du phosphate de potassium, du chlorure de sodium, de la lysine et de l'arginine, et ajusté à un pH de 6,1 à 6,9.

12. Procédé selon l'une quelconque des revendications 1 à 11, caractérisé en ce que le Facteur XI est élué par une augmentation du pH du tampon à une valeur comprise entre 7 et 8 et de la quantité de chlorure de sodium à une concentration comprise entre 0,15 et 0,20 M.

13. Procédé selon l'une quelconque des revendications 1 à 12, caractérisé en ce que la solution de Facteur XI éluée est stabilisée par l'addition de 0,5 à 3 U/ml d'antithrombine III et de 0,5 à 4 U/ml d'héparine par 100 U de Facteur XI et immédiatement conditionnée et lyophilisée.

## Claims

1. Process for preparing a Factor XI concentrate of suitable quality for therapeutic use, characterized in that it includes a filtration-adsorption step and a single step of chromatography on cation exchange resin.

2. Process according to claim 1, characterized in that the filtration-adsorption step is conducted on a series of filter cartridges having a porosity of 0.5 to 2µ, composed of cellulose and perlites and negatively charged, as well as a small quantity of positively charged resin.

3. Process according to claim 1 or 2, characterized in that the filter is rinsed with buffer solution including sodium citrate, disodium phosphate, sodium chloride and disodium EDTA, adjusted to a pH of 5.5 to 6.5.

4. Process according to any one of claims 2 to 3, characterized in that the Factor XI is desorbed from the filters by an increase in the ionic strength of the buffer solution.

5. Process according to claim 4, characterized in that the ionic strength of the buffer solution is increased by increasing the sodium chloride concentration to 0.5 to 2 M.

6. Process according to claim 5, characterized in that 0.1 to 0.2 U/ml of antithrombin III is added to the elution buffer.

7. Process according to any one of claims 2 to 6, characterized in that the desorbed solution of Factor XI is dialyzed, concentrated and subjected to a viral inactivation treatment.

8. Process according to any one of claims 2 to 7, characterized in that the Factor XI solution is subjected to a chromatography on a cation exchange resin.

9. Process according to claim 8, characterized in that the resin is sepharose sulphate.

10. Process according to any one of claims 2 to 9, characterized in that the chromatography column is equilibrated with buffer solution including sodium citrate, sodium chloride, lysine and arginine and adjusted to a pH of 5.5 to 6.5.

11. Process according to any one of claims 2 to 10, characterized in that, after the column has been loaded with the Factor XI solution, it is rinsed using buffer solution including sodium citrate, disodium phosphate, potassium phosphate, sodium chloride, lysine, and arginine, and adjusted to a pH of 6.1 to 6.9.

12. Process according to any one of claims 2 to 11, characterized in that the Factor XI is eluted by increasing the pH of the buffer to a value of between 7 and 8 and the quantity of sodium chloride to a concentration of between 0.15 to 0.20 M.

13. Process according to any one of claims 2 to 12, characterized in that the eluted Factor XI solution is stabilized by the addition of 0.5 to 3 U/ml of antithrombin III and 0.5 to 5 U/ml of heparin per 100 U of Factor XI, and is immediately dispensed and freeze dried.

## Patentansprüche

1. Verfahren zur Herstellung eines Konzentrates des menschlichen Faktors XI mit für therapeutische Anwendungen geeigneter Reinheit, dadurch gekennzeichnet, daß es einen Filtrations-Adsorptions-Schritt aufweist sowie einen Einzelschritt mit Chromatographie auf einem Kationenaustauscherharz.

2. Verfahren nach Anspruch 1, dadurch gekennzeichnet, daß der Filtrations-Adsorptions-Schritt auf einer Reihe von Kartuschen mit Filtern der Porosität 0,5 bis 2 µ, die aus Cellulose und Perliten zusammengesetzt sind und negative Ladungen tragen, sowie aus einer kleinen Menge eines positiv geladenen Harzes, erfolgt.

3. Verfahren nach Anspruch 1 oder 2, dadurch gekennzeichnet, daß der Filter mit einem auf einen pH-Wert von 5,5 bis 6,5 eingestellten Puffer gespült wird, der Natriumcitrat, Dinatriumphosphat, Kaliumphosphat, Natriumchlorid und Dinatrium-EDTA enthält.

4. Verfahren nach einem der Ansprüche 1 bis 3, dadurch gekennzeichnet, daß der Faktor XI durch Anheben der Ionenstärke des Puffers von den Filtern desorbiert wird.

5. Verfahren nach Anspruch 4, dadurch gekennzeichnet, daß die Ionenstärke des Puffers erhöht wird, indem die Natriumchloridkonzentration auf 0,5 bis 2 M gebracht wird.

6. Verfahren nach Anspruch 5, dadurch gekennzeichnet, daß 0,1 bis 0,2 E/ml an Antithrombin III dem Elutionspuffer zugesetzt werden.

7. Verfahren nach einem der Ansprüche 1 bis 6, dadurch gekennzeichnet, daß die desorbierte Lösung des Faktors XI dialysiert, konzentriert und einer Behandlung zur Virusinaktivierung unterzogen wird.

8. Verfahren nach einem der Ansprüche 1 bis 7, dadurch gekennzeichnet, daß die Lösung des Faktors XI einer Chromatographie auf einem Kationenaustauscherharz unterzogen wird.

9. Verfahren nach Anspruch 8, dadurch gekennzeichnet, daß das Harz aus Sepharose-Sulfat besteht.

10. Verfahren nach einem der Ansprüche 1 bis 9, dadurch gekennzeichnet, daß die Chromatographiesäule mit einem auf einen pH-Wert von 5,5 bis 6,5 eingestellten Puffer mit Natriumcitrat, Natriumchlorid, Lysin und Arginin equilibriert wird.

11. Verfahren nach einem der Ansprüche 1 bis 10, dadurch gekennzeichnet, daß nach der Beschickung der Säule mit der Lösung des Faktors XI diese mit einem auf einen pH-Wert von 6,1 bis 6,9 eingestellten Puffer gespült wird, der Natriumcitrat, Dinatriumphosphat, Kaliumphosphat, Natriumchlorid, Lysin und Arginin enthält.

12. Verfahren nach einem der Ansprüche 1 bis 11, dadurch gekennzeichnet, daß der Faktor XI eluiert wird, indem der pH-Wert des Puffers auf einen Wert zwischen 7 und 8 und die Menge an Natriumchlorid auf 0,15 bis 0,20 M angehoben werden.

13. Verfahren nach einem der Ansprüche 1 bis 12, dadurch gekennzeichnet, daß die eluierte Lösung des Faktors XI durch Zugabe von 0,5 bis 3 E /ml an Antithrombin III und von 0,5 bis 4 E /ml an Heparin pro 100 Einheiten des Faktors XI stabilisiert und unmittelbar darauf konditioniert und lyophilisiert wird.
